# EUROPÄISCHE PATENTSCHRIFT

(11) **EP 1 968 746 B1**
(45) Veröffentlichungstag und Bekanntmachung des Hinweises auf die Patenterteilung: **11.08.2010**
(21) Anmeldenummer: 06830534.1
(22) Anmeldetag: 12.12.2006
(51) Int. Cl.: B01J 31/10, B01J 20/26, B01J 31/16

(54) **SAUER FUNKTIONALISIERTE METALLORGANISCHE GERÜSTMATERIALIEN**
ACID-FUNCTIONALIZED METALORGANIC FRAMEWORKS
MATERIAUX DE STRUCTURE METALORGANIQUE A FONCTIONNALISATION ACIDE

(30) Priorität: 16.12.2005 DE 102005060364
(43) Veröffentlichungstag der Anmeldung: 17.09.2008
(73) Patentinhaber: BASF SE, 67056 Ludwigshafen (DE)
(72) Erfinder: SCHUBERT, Markus, 67063 Ludwigshafen (DE); MÜLLER, Ulrich, 67435 Neustadt (DE); BOSCH, Marco, 2000 Antwerpen (BE); TRILLER, Michael, 68549 Mannheim (DE); HATSCHER, Stephan, 28857 Syke (DE)
(86) Internationale Anmeldenummer: PCT/EP2006/069568
(87) Internationale Veröffentlichungsnummer: WO 2007/068681

(56) Entgegenhaltungen:
- WO-A2-2004/084834
- US-A1- 2004 081 611
- WAN, SHUANG-YI ET AL: "Synthesis, structure and anion - exchange property of the first example of self-penetrated three-dimensional metal - organic framework with flexible three-connecting ligand and nickel(II) perchlorate" MICROPOROUS AND MESOPOROUS MATERIALS ( 2004 ), 73(1-2), 101-108 CODEN: MIMMFJ; ISSN: 1387-1811, 2003, XP002422631

## Beschreibung

Die Erfindung betrifft metallorganische Gerüstmaterialien, Verfahren zu deren Herstellung sowie deren Verwendung.

Feststoffe mit sauren Eigenschaften sind für zahlreiche Anwendungen vorteilhaft. Eine dieser Anwendungen stellt die lonenaustausch-Chromatographie dar. Hierbei enthalten die festen Stoffe, welche üblicherweise Ionenaustauscher genannt werden, einen Anteil, der in den austauschaktiven Gruppen gebundene Ionen reversibel gegen andere Ionen austauschen können.

Die Ionenaustauscher werden je nach Ladung des austauschbaren Ions in Kationenaustauscher und Anionenaustauscher unterschieden. Kationenaustauscher, wie sie im Stand der Technik bekannt sind, sind meist aus einem hochmolekularen polyvalenten Anion mit beweglichen Kationen, wie zum Beispiel einer Hydroxygruppe, einer Sulfonsäuregruppe, einer Carboxygruppe oder einer Phosphonsäuregruppe als austauschaktive Gruppe, aufgebaut. Um den Austausch besonders effizient zu gestalten, sind als Feststoffe insbesondere makroporöse Harze von Interesse, die mitunter Porenweiten bis zu 10 nm aufweisen können. Zur Herstellung der Ionenaustauscher werden typischerweise die funktionellen Gruppen in Polykondensationsharze und Polymerisationsharze eingeführt. Herkömmliche stark saure Ionenaustauscher können beispielsweise auf Basis von Styrol/Divinylbenzolcopolymeren durch Suspensionspolymerisation und anschließende Sulfonierung erhalten werden. Bei handelsüblichen Ionenaustauschern handelt es sich meist um kugelförmige Partikel mit einer Größe von etwa 0,3 bis 1,2 mm. Beispiele von Ionenaustauschern sind unter den Markennamen Dowex^{®} (Fa. Dow), Amberlite^{®}, Amberjet^{®} und Amberlyst^{®} (jeweils Fa. Rohm & Haas) und Lewatit K^{®} (Fa. Lanxess) erhältlich.

Insbesondere für katalytische Anwendungen ist es wichtig, dass die sulfonierten Polymermatrices eine wie oben aufgeführte Porenstruktur aufweisen, die eine Diffusion der Reaktanden zu und von den austauschaktiven Gruppen erlauben. Makroporöse Ionenaustauscher werden beispielsweise in US-A 5,231,115 und US-B 6,329,435 beschrieben. Hierbei wird durch die Zugabe von Additiven wie gesättigten Kohlenwasserstoffen, gesättigten Alkoholen und/oder wasserlöslichen Polymeren während der Polymerisation ein Aufquellen der eigentlichen Styrol/Divinylbenzol-Polymermatrix erreicht, um somit eine porenähnliche Struktur erhalten zu können. Um eine ausreichende mechanische Stabilität der makroporösen Polymermatrix zu gewährleisten, muss dabei der Anteil an quervemetzendem Monomer (zum Beispiel Divinylbenzol) erhöht werden.

Die anschließende Sulfonierung ermöglicht die Derivatisierung des Copolymergerüstes durch Sulfonsäuregruppen. Hierbei werden die vorhandenen Phenylgruppen durch elektrophile Substitution am Aromaten mit Sulfonsäuregruppen versehen. Ionenaustauscher auf Basis von Styrol/Divinylbenzol-Matrices, die neben Sulfonsäuregruppen auch Phosphonsäuregruppen enthalten, sind beispielsweise in US-B 6,488,859 beschrieben.

In jüngerer Zeit wurden metallorganische Gerüstmaterialien beschrieben, die ebenfalls wie die oben erwähnten Polymere durch ihre Porosität auffallen, Die porösen metallorganischen Gerüstmaterialien enthalten typischerweise mindestens eine an mindestens ein Metallion koordinativ gebundene, mindestens zweizähnige organische Verbindung, die meist eine Di-, Tri- oder Tetracarbonsäure darstellt. Solche metallorganischen Gerüstmaterialien (MOF = metal organic framework) werden beispielsweise in US-A 5,648,508, EP-A 0 790 253, M. O. Keeffe, J. Sol. State Chem. 152 (2000), 2-20; H. P. Li et al., Nature 402 (1999), 276; M. Eddaoudi, Topics in catalysis 9 (1999), 105-111; B. Chen et al., Science 91 (2001), 1021-1023 und DE-A 101 11 230 beschrieben.

Obwohl die porösen metallorganischen Gerüstmaterialien Carbonsäuren enthalten, weisen diese typischerweise keine sauren Eigenschaften auf. Dies liegt daran, dass die Carbonsäuren in Form ihrer Carboxylate am Gerüstaufbau beteiligt sind, wobei die Carboxylate entsprechend koordinativ an das jeweilige Metall gebunden sind und somit nicht als saure austauschaktive Gruppe zur Verfügung stehen.

Es wurden auch poröse metallorganische Gerüstmaterialien publiziert, die die insbesondere für Kationenaustauscher interessanten funktionellen Gruppen, nämlich Sulfonat und Phosphonat, aufweisen. So offenbart ES-A 2 200 681 Seltenerd-Disulfonate und R. Fu et al. beschreiben in Euro. J. Inorg. Chem. 2005, 3211-3213 Gerüstmaterialien, die Phosphonatgruppen enthalten.

Bei beiden Publikationen wird jedoch wie oben ausgeführt die saure funktionelle Gruppe zum Aufbau des Gerüstmaterials eingesetzt. Freie austauschaktive Gruppen stehen daher nicht zur Verfügung, so dass auch diese porösen metallorganischen Gerüstmaterialien nicht beispielsweise als Ionenaustauscher geeignet sind.

Es besteht daher ein Bedarf, sauer funktionalisierte metallorganische Gerüstmaterialien bereitzustellen, die beispielsweise als Ionenaustauscher eingesetzt werden können und so die vorteilhaften Eigenschaften metallorganischer Gerüstmaterialien in Anwendungen, die saure poröse Polymere erfordern oder vorteilhaft erscheinen lassen, besitzen.

Diese Aufgabe wird gelöst durch ein poröses metallorganisches Gerüstmaterial enthaltend mindestens eine an mindestens ein Metallion M koordinativ gebundene, mindestens zweizähnige organische Verbindung L, wobei L mindestens eine funktionelle Gruppe G aufweist, die nicht koordinativ an M bindet und ausgewählt ist aus der Gruppe bestehend aus -SO₃H und deren deprotoniertes Analogon, wobei das Gerüstmaterial eine Säuredichte von mindestens 0,1 mmol/g Gerüstmaterial aufweist.

Es wurde nämlich überraschenderweise gefunden, dass durch die Modifizierung von an sich bekannten porösen metallorganischen Gerüstmaterialien durch die funktionelle Gruppe G neue poröse metallorganische Gerüstmaterialien erhalten werden, die sich durch ihre sauren Eigenschaften sowie deren Verwendung beispielsweise als Ionenaustauscher auszeichnen.

Das deprotonierte Analogon der Gruppe G ist -SO₃⁻. Vorzugsweise liegen jedoch mindestens 50 % der Gruppe G in protonierter Form vor, mehr bevorzugt mindestens 75 % und am meisten bevorzugt liegt die Gruppe G in vollständig protonierter Form vor. Für den Fall, dass G zumindest teilweise in deprotonierter Form vorliegt, sind Alkalimetallionen sowie Ammoniumionen geeignete Gegenionen.

Zum Aufbau des Gerüstmaterials sollte ein Metallion M von mindestens zwei Molekülen der Verbindung L koordinativ gebunden sein.

In dem porösen metallorganischen Gerüstmaterial beträgt vorzugsweise das molare Verhältnis G : M mindestens 1 : 75. Mehr bevorzugt beträgt dieses mindestens 1 : 50, weiterhin mehr bevorzugt beträgt das Verhältnis mindestens 1 : 10.

Vorzugsweise beträgt das molare Verhältnis G : M höchstens 4 : 1, mehr bevorzugt höchstens 2 : 1 und besonders bevorzugt höchstens 1 : 1.

Das entsprechende Verhältnis G : M beziehungsweise L : M kann durch entsprechende Reaktionsführung bei der Herstellung des erfindungsgemäßen porösen metallorganischen Gerüstmaterials in gewünschter Weise eingestellt werden. Dies kann durch für den Fachmann bekannte Methoden erfolgen und hängt von dem entsprechenden Herstellverfahren ab. So kann zum Beispiel bei der Herstellung die organische Verbindung L die funktionelle Gruppe G oder eine analoge Gruppe aufweisen, die in G überführt werden kann. Zur Einstellung des gewünschten molaren Verhältnisses G : M kann bei der Umsetzung der Verbindung L mit M weiterhin eine organische Verbindung L' eingesetzt werden, die wie L aufgebaut ist, jedoch nicht G oder ein Derivat von G aufweist. Aufgrund des Mischungsverhältnisses von L zu L' kann bei der Umsetzung mit M das oben genannte molare Verhältnis G : M entsprechend eingestellt werden. Eine weitere Möglichkeit zur Einstellung eines bestimmten molaren Verhältnisses liegt darin, dass die Gruppe G nachträglich, d.h. nachdem bereits ein metallorganisches Gerüstmaterial gebildet wurde, eingeführt wird. Dies kann beispielsweise durch Sulfonierung eines Aromaten geschehen. In diesem Fall kann das molare Verhältnis G : M durch die Einwirkdauer, Temperatur und Konzentration des Sulfonierungsreagenzes gesteuert werden.

Es existieren zahlreiche Methoden zur Bestimmung des molaren Verhältnisses, die an sich dem Fachmann bekannt sind. Das Verhältnis kann über gängige Methoden wie Kernresonanzspektroskopie, Infrarotspektroskopie, Thermodesorption von zum Beispiel Aminen, Elementaranalyse und/oder Titration festgestellt werden.

Der Gehalt der Gruppe G im porösen metallorganischen Gerüstmaterial bestimmt auch die Säureeigenschaften des erfindungsgemäßen Gerüstmaterials. Dieses weist eine Säuredichte von mindestens 0,1 mmol/g auf. Vorzugsweise beträgt die Säuredichte mindestens 1 mmol/g, mehr bevorzugt mindestens 2 mmol/g.

Die Metallkomponente im Gerüstmaterial nach der vorliegenden Erfindung ist vorzugsweise ausgewählt aus den Gruppen Ia, IIa, IIIa, IVa bis VIIIa und Ib bis VIb. Besonders bevorzugt sind Mg, Ca, Sr, Ba, Sc, Y, Ti, Zr, Hf, V, Nb, Ta, Cr, Mo, W, Mn, Re, Fe, Ro, Os, Co, Rh, Ir, Ni, Pd, Pt, Cu, Ag, Au, Zn, Cd, Hg, Al, Ga, In, TI, Si, Ge, Sn, Pb, As, Sb und Bi. Mehr bevorzugt sind Zn, Cu, Ni, Pd, Pt, Ru, Rh und Co. Insbesondere bevorzugt Zn, Al, Ni und Cu. In Bezug auf die Ionen dieser Elemente sind besonders zu erwähnen Mg²⁺, Ca²⁺, Sr²⁺, Ba²⁺, Sc³⁺, Y³⁺, Ti⁴⁺, Zr⁴⁺, Hf⁴⁺, V⁴⁺, V³⁺, V²⁺, Nb³⁺, Ta³⁺, Cr³⁺, Mo³⁺, W³⁺, Mn³⁺, Mn²⁺, Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga³⁺, In³⁺, TI³⁺, Si⁴⁺, Si²⁺, Ge⁴⁺, Ge²⁺, Sn⁴⁺, Sn²⁺, Pb⁴⁺, Pb²⁺, As⁵⁺, As³⁺, As⁺, Sb⁵⁺, Sb³⁺, Sb⁺, Bi⁵⁺, Bi³⁺ und Bi⁺.

Mehr bevorzugt sind die Metalle Sr, Ba, Mo, W, V, Ni, Co, Se, Y, Platin- und Seltenerdmetalle sowie Mg, Ca, Al, Ga, In, Zn, Cu, Fe und Mn.

Besonders bevorzugt ist M ausgewählt aus der Gruppe bestehend aus Mg, Ca, Al, Ga, In, Zn, Cu, Fe und Mn. Insbesondere bevorzugt sind Mg, Al.

Der Begriff "mindestens zweizähnige organische Verbindung" bezeichnet eine organische Verbindung, die mindestens eine funktionelle Gruppe enthält, die in der Lage ist, zu einem gegebenen Metallion mindestens zwei, bevorzugt zwei koordinative Bindungen, und/oder zu zwei oder mehr, bevorzugt zwei Metallatomen jeweils eine koordinative Bindung auszubilden.

Als funktionelle Gruppen, über die die genannten koordinativen Bindungen ausgebildet werden kann, sind insbesondere beispielsweise folgende funktionellen Gruppen zu nennen: OH, SH, NH₂, NH(-R-H), N(R-H)₂, CH₂OH, CH₂SH, CH₂NH₂, CH₂NH(-R-H), CH₂N(-R-H)₂, -CO₂H, COSH, -CS₂H, -NO₂, -B(OH)₂, -SO₃H, -Si(OH)₃, -Ge(OH)₃, -Sn(OH)₃, -Si(SH)₄, -Ge(SH)₄, -Sn(SH)₃, -PO₃H₂, -AsO₃H, -AsO₄H, -P(SH)₃, -As(SH)₃, -CH(RSH)₂, -C(RSH)₃, -CH(RNH₂)₂, -C(RNH₂)₃, -CH(ROH)₂, -C(ROH)₃, -CH(RCN)₂, - C(RCN)₃, wobei R beispielsweise bevorzugt eine Alkylengruppe mit 1, 2, 3, 4 oder 5 Kohlenstoffatomen wie beispielsweise eine Methylen-, Ethylen-, n-Propylen-, i-Propylen, n-Butylen-, i-Butylen-, tert-Butylen- oder n-Pentylengruppe, oder eine Arylgruppe, enthaltend 1 oder 2 aromatische Kerne wie beispielsweise 2 C₆-Ringe, die gegebenenfalls kondensiert sein können und unabhängig voneinander mit mindestes jeweils einem Substituenten geeignet substituiert sein können, und/oder die unabhängig voneinander jeweils mindestens ein Heteroatom wie beispielsweise N, O und/oder S enthalten können. Gemäß ebenfalls bevorzugter Ausführungsformen sind funktionelle Gruppen zu nennen, bei denen der oben genannte Rest R nicht vorhanden ist. Diesbezüglich sind unter anderem -CH(SH)₂, -C(SH)₃, -CH(NH₂)₂, CH(NH(R-H))₂, CH(N(R-H)₂)₂, C(NH(R-H))₃, C(N(R-H)₂)₃, -C(NH₂)₃, -CH(OH)₂, -C(OH)₃, -CH(CN)₂, -C(CN)₃ zu nennen.

Vorzugsweise wird die koordinative Bindung nicht über -SO₃H und/oder PO₃H₂ ausgebildet.

Die mindestens zwei funktionellen Gruppen können grundsätzlich an jede geeignete organische Verbindung gebunden sein, solange gewährleistet ist, dass die diese funktionellen Gruppen aufweisende organische Verbindung zur Ausbildung der koordinativen Bindung und zur Herstellung des Gerüstmaterials befähigt ist.

Bevorzugt leiten sich die organischen Verbindungen, die die mindestens zwei funktionellen Gruppen enthalten, von einer gesättigten oder ungesättigten aliphatischen Verbindung oder einer aromatischen Verbindung oder einer sowohl aliphatischen als auch aromatischen Verbindung ab.

Die aliphatische Verbindung oder der aliphatische Teil der sowohl aliphatischen als auch aromatischen Verbindung kann linear und/oder verzweigt und/oder cyclisch sein, wobei auch mehrere Cyclen pro Verbindung möglich sind. Weiter bevorzugt enthält die aliphatische Verbindung oder der aliphatische Teil der sowohl aliphatischen als auch aromatischen Verbindung 1 bis 16, weiter bevorzugt 1 bis 14, weiter bevorzugt 1 bis 13, weiter bevorzugt 1 bis 12, weiter bevorzugt 1 bis 11 und insbesondere bevorzugt 1 bis 10 C-Atome wie beispielsweise 1, 2, 3, 4, 5, 6, 7, 8, 9 oder 10 C-Atome. Insbesondere bevorzugt sind hierbei unter anderem Methan, Adamantan, Acetylen, Ethylen oder Butadien.

Die aromatische Verbindung oder der aromatische Teil der sowohl aromatischen als auch aliphatischen Verbindung kann einen oder auch mehrere Kerne wie beispielsweise zwei, drei, vier oder fünf Kerne aufweisen, wobei die Kerne getrennt voneinander und/oder mindestens zwei Kerne in kondensierter Form vorliegen können. Besonders bevorzugt weist die aromatische Verbindung oder der aromatische Teil der sowohl aliphatischen als auch aromatischen Verbindung einen, zwei oder drei Kerne auf, wobei einer oder zwei Kerne besonders bevorzugt sind. Unabhängig voneinander kann weiter jeder Kern der genannten Verbindung mindestens ein Heteroatom wie beispielsweise N, O, S, B, P, Si, Al, bevorzugt N, O und/oder S enthalten. Weiter bevorzugt enthält die aromatische Verbindung oder der aromatische Teil der sowohl aromatischen als auch aliphatischen Verbindung einen oder zwei C₆-Kerne, wobei die zwei entweder getrennt voneinander oder in kondensierter Form vorliegen. Insbesondere sind als aromatische Verbindungen Benzol, Naphthalin und/oder Biphenyl und/oder Bipyridyl und/oder Pyridyl zu nennen.

Besonders bevorzugt leitet sich L von einer Di-, Tri-, Tetracarbonsäure oder einem Schwefelanalogon oder einem Diamin ab. Schwefelanaloga sind die funktionellen Gruppen -C(=O)SH sowie dessen Tautomer und C(=S)SH. Die Carbonsäure oder das Diamin können neben den funktionellen Gruppen, die gemeinsam mit dem Metall M das Gerüst bilden, weitere Substituenten aufweisen, die nach deren Umwandlung die Gruppe G ergeben. Zudem können weitere Substituenten vorhanden sein. Solche Substituenten sind beispielsweise -OH, -NH₂, -SH, -NO₂, Halogene, wie Fluor, Chlor, Brom oder Iod sowie Pseudohalogenide wie -CN, -CNO, -CNS oder Alkyl- oder Alkoxygruppen mit 1 bis 4 Kohlenstoffatomen, wie Methoxy oder Ethoxy. Die Gruppe G kann auch über solche Substituenten an L gebunden sein. Es ist daher nicht erforderlich, dass G an das Grundgerüst von L gebunden ist. Wie bereits oben ausgeführt wurde, muss nicht jede mindestens zweizähnige organische Verbindung, die bei dem Gerüstaufbau beteiligt ist, eine Gruppe G aufweisen. In einem solchen Falle ist es jedoch bevorzugt, dass die mindestens zweizähnige organische Verbindung, die verschieden von L ist, sich lediglich im Vorhandensein der Gruppe G von L unterscheidet.

Bevorzugte Diamine sind 1,4-Phenylendiamin, 1,2-Phenylendiamin, 1,3-Phenylendiamin, 1,2-Cyclohexandiamin, 1,3-Cyclohexandiamin, 1,4-Cyclohexandiamin, 3,6-Diazaoctan-1,8-diamin, Diethylendiamin, Ethylendiamin, Propylendiamin, Trimethylendiamin, 1,1'-Biphenyl-4,4'-diamin, 1,7-Heptandiamin, Isophorondiamin, 2-Methylpentamethylendiamin, 4-Methyl-1,2-phenyldiamin, 4-Methyl-1,3-phenylendiamin, Naphthalin-1,5-diamin, Naphthalin-1,8-diamin, Neopentandiamin, 2-Nitro-1,4-phenylendiamin, 4-Nitro-1,2-phenylendiamin, 4-Nitro-1,3-phenylendiamin, Nonamethylendiamin, 1,3-Propandiamin, Triethylendiamin (DABCO) 3,5-Diaminobenzoesäure, 3,4-Diaminobenzoesäure, 4,4'-Diaminobenzophenon, 1,4-Diaminobutan, 2,4-Diamino-6-chlorpyrimidin, 2,2'-Diaminodiethylamin, 1,8-Diamino-3,6-dioxaoctan, 4,4'-Diaminodiphenylether, 3,3'-Diaminodiphenylsulfon, 4,4'-Diaminodiphenylsulfon, 1,6-Diaminohexan, 4,5-Diamino-6-hydroxy-2-mercaptopyridin, 2,4-Diamino-6-hydroxypyrimidin, Diaminomaleinsäuredinitril, 4,6-Diamino-2-mercaptopyrimidin, 1,5-Diamino-2-methylpentan, 1,9-Diaminononan, 1,8-Diaminooctan, 2,4-Diaminophenol, 2,6-Diamino-4-phenyl-1,3,5-triazin, 2,3-Diaminopyridin, 2,6-Diamonopyridin, 2,3-Diaminopropionsäure, 3,4-Diaminopyridin, 4,6-Diamino-2-pyrimidinthiol, 3,5-Diamino-1,2,4-triazol, 1,13-Diamino-4,7,10-trioxatridecan sowie 2,5-Diaminovaleriansäure.

Beispielsweise sind im Rahmen der vorliegenden Erfindung Dicarbonsäuren wie etwa
Oxalsäure, Bernsteinsäure, Weinsäure, 1,4-Butandicarbonsäure, 1,4-Butendicarbonsäure, 4-Oxo-Pyran-2,6-dicarbonsäure, 1,6-Hexandicarbonsäure, Decandicarbonsäure, 1,8-Heptadecandicarbonsäure, 1,9-Heptadecandicarbonsäure, Heptadecandicarbonsäure, Acetylendicarbonsäure, 1,2-Benzoldicarbonsäure, 1,3-Benzoldicarbonsäure, 2,3-Pyridindicarbonsäure, Pyridin-2,3-dicarbonsäure, 1,3-Butadien-1,4-dicarbonsäure, 1,4-Benzoldicarbonsäure, p-Benzoldicarbonsäure, Imidazol-2,4-dicarbonsäure, 2-Methylchinolin-3,4-dicarbonsäure, Chinolin-2,4-dicarbonsäure, Chinoxalin-2,3-dicarbonsäure, 6-Chlorchinoxalin-2,3-dicarbonsäure, 4,4'-Diaminphenylmethan-3,3'-dicarbonsäure, Chinolin-3,4-dicarbonsäure, 7-Chlor-4-hydroxychinolin-2,8-dicarbonsäure, Diimiddicarbonsäure, Pyridin-2,6-dicarbonsäure, 2-Methylimidazol-4,5-dicarbonsäure, Thiophen-3,4-dicarbonsäure, 2-Isopropylimidazol-4,5-dicarbonsäure, Tetrahydropyran-4,4-dicarbonsäure, Perylen-3,9-dicarbonsäure, Perylendicarbonsäure, Pluriol E 200-dicarbonsäure, 3,6-Dioxaoctandicarbonsäure, 3,5-Cyclohexadien-1,2-dicarbonsäure, Octadicarbonsäure, Pentan-3,3-carbonsäure, 4,4'-Diamino-1,1'-diphenyl-3,3'-dicarbonsäure, 4,4'-Diaminodiphenyl-3,3'-dicarbonsäure, Benzidin-3,3'-dicarbonsäure, 1,4-Bis-(phenylamino)-benzol-2,5-dicarbonsäure, 1,1'-Dinaphthyldicarbonsäure, 7-Chlor-8-methylchinolin-2,3-dicarbonsäure, 1-Anilinoanthrachinon-2,4'-dicarbonsäure, Polytetrahydrofuran-250-dicarbonsäure, 1,4-Bis-(carboxymethyl)-piperazin-2,3-dicarbonsäure, 7-Chlorchinolin-3,8-dicarbonsäure, 1-(4-Carboxy)-phenyl-3-(4-chlor)-phenylpyrazolin-4,5-dicarbonsäure, 1,4,5,6,7,7,-Hexachlor-5-norbomen-2,3-dicarbonsäure, Phenylindandicarbonsäure, 1,3-Dibenzyl-2-oxo-imidazolidin-4,5-dicarbonsäure, 1,4-Cyclohexandicarbonsäure, Naphthalin-1,8-dicarbonsäure, 2-Benzoylbenzol-1,3-dicarbonsäure, 1,3-Dibenzyl-2-oxoimidazolidin-4,5-cis-dicarbonsäure, 2,2'-Bichinolin-4,4'-dicarbonsäure, Pyridin-3,4-dicarbonsäure, 3,6,9-Trioxaundecandicarbonsäure, Hydroxybenzophenon-dicarbonsäure, Pluriol E 300-dicarbonsäure, Pluriol E 400-dicarbonsäure, Pluriol E 600-dicarbonsäure, Pyrazol-3,4-dicarbonsäure, 2,3-Pyrazindicarbonsäure, 5,6-Dimethyl-2,3-pyrazindicarbonsäure, 4,4'-Diaminodiphenyletherdümiddicarbonsäure, 4,4'-Diaminodiphenylmethandiimiddicarbonsäure, 4,4'-Diaminodiphenylsulfondiimiddicarbonsäure, 1,4-Naphthalindicarbonsäure, 2,6-Naphthalindicarbonsäure, 1,3-Adamantandicarbonsäure, 1,8-Naphthalindicarbonsäure, 2,3-Naphthalindicarbonsäure, 8-Methoxy-2,3-naphthalindicarbonsäure, 8-Nitro-2,3-naphthalindicarbönsäure, 8-Sulfo-2,3-naphthalindicarbonsäure, Anthracen-2,3-dicarbonsäure, 2',3'-Diphenyl-p-terphenyl-4,4"-dicarbonsäure, Diphenylether-4,4'-dicarbonsäure, Imidazol-4,5-dicarbonsäure, 4(1H)-Oxothiochromen-2,8-dicarbonsäure, 5-tert-Butyl-1,3-benzoldicarbonsäure, 7,8-Chinolindicarbonsäure, 4,5-Imidazoldicarbonsäure, 4-Cyclohexen-1,2-dicarbonsäure, Hexatriacontandicarbonsäure, Tetradecandicarbonsäure, 1,7-Heptadicarbonsäure, 5-Hydroxy-1,3-Benzoldicarbonsäure, 2,5-Dihydroxy-1,4-Benzoldicarbonsäure, Pyrazin-2,3-dicarbonsäure, Furan-2,5-dicarbonsäure, 1-Nonen-6,9-dicarbonsäure, Eicosendicarbonsäure, 4,4'-Dihydroxydiphenylmethan-3,3'-dicarbonsäure, 1-Amino-4-methyl-9,10-dioxo-9,10-dihydroanthracen-2,3-dicarbonsäure, 2,5-Pyridindicarbonsäure, Cyclohexen-2,3-dicarbonsäure,2,9-Dichlorfluorubin-4,11-dicarbonsäure, 7-Chlor-3-methylchinolin-6,8-dicarbonsäure, 2,4-Dichlorbenzophenon-2',5'-dicarbonsäure, 1,3-Benzoldicarbonsäure, 2,6-Pyridindicarbonsäure, 1-Methylpyrrol-3,4-dicarbonsäure, 1-Benzyl-1H-pyrrol-3,4-dicarbonsäure, Anthrachinon-1,5-dicarbonsäure, 3,5-Pyrazoldicarbonsäure, 2-Nitrobenzol-1,4-dicarbonsäure, Heptan-1,7-dicarbonsäure, Cyclobutan-1,1-dicarbonsäure 1,14-Tetradecandicarbonsäure, 5,6-Dehydronorbornan-2,3-dicarbonsäure, 5-Ethyl-2,3-pyridindicarbonsäure oder Campherdicarbonsäure,
Tricarbonsäuren wie etwa
2-Hydroxy-1,2,3-propantricarbonsäure, 7-Chlor-2,3,8-chinolintricarbonsäure, 1,2,3-, 1,2,4-Benzoltricarbonsäure, 1,2,4-Butantricarbonsäure, 2-Phosphono-1,2,4-butantricarbonsäure, 1,3,5-Benzoltricarbonsäure, 1-Hydroxy-1,2,3-Propantricarbonsäure, 4,5-Dihydro-4,5-dioxo-1H-pyrrolo[2,3-F]chinolin-2,7,9-tricarbonsäure, 5-Acetyl-3-amino-6-methylbenzol-1,2,4-tricarbonsäure, 3-Amino-5-benzoyl-6-methylbenzol-1,2,4-tricarbon-+säure, 1,2,3-Propantricarbonsäure oder Aurintricarbonsäure,
oder Tetracarbonsäuren wie etwa
1,1-Dioxidperylo[1,12-BCD]thiophen-3,4,9,10-tetracarbonsäure, Perylentetracarbonsäuren wie Perylen-3,4,9,10-tetracarbonsäure oder oder Perylen-1,12-sulfon-3,4,9,10-tetracarbonsäure, Butantetracarbonsäuren wie 1,2,3,4-Butantetracarbonsäure oder Meso-1,2,3,4-Butantetracarbonsäure, Decan-2,4,6,8-tetracarbonsäure, 1,4,7,10,13,16-Hexaoxacyclooctadecan-2,3,11,12-tetracarbonsäure, 1,2,4,5-Benzoltetracarbonsäure, 1,2,11,12-Dodecantetracarbonsäure, 1,2,5,6-Hexan-tetracarbonsäure, 1,2,7,8-Octantetracarbonsäure, 1,4,5,8-Naphthalintetracarbonsäure, 1,2,9,10-Decantetracarbonsäure, Benzophenontetracarbonsäure, 3,3',4,4'-Benzo-phenontetracarbonsäure, Tetrahydrofurantetracarbonsäure oder Cyclopentantetracarbonsäuren wie Cyclopentan-1,2,3,4-tetracarbonsäure zu nennen.

Ganz besonders bevorzugt werden gegebenenfalls mindestens einfach substituierte mono-, di-, tri-, tetra- oder höherkernige aromatische Di-, Tri- oder Tetracarbonsäuren eingesetzt, wobei jeder der Kerne mindestens ein Heteroatom enthalten kann, wobei zwei oder mehr Kerne gleiche oder unterschiedliche Heteroatome enthalten können. Beispielsweise bevorzugt werden monokernige Dicarbonsäuren, monokernige Tricarbonsäuren, monokernige Tetracarbonsäuren, dikernige Dicarbonsäuren, dikernige Tricarbonsäuren, dikernige Tetracarbonsäuren, trikernige Dicarbonsäuren, trikernige Tricarbonsäuren, trikernige Tetracarbonsäuren, tetrakernige Dicarbonsäuren, tetrakernige Tricarbonsäuren und/oder tetrakernige Tetracarbonsäuren. Geeignete Heteroatome sind beispielsweise N, O, S, B, P, Si, bevorzugte Heteroatome sind hierbei N, S und/oder O. Als geeigneter Substituent ist diesbezüglich unter anderem -OH, eine Nitrogruppe, eine Aminogruppe oder eine Alkyl- oder Alkoxygruppe zu nennen.

Insbesondere bevorzugt werden als mindestens zweizähnige organische Verbindungen Acetylendicarbonsäure (ADC), Campherdicarbonsäure, Fumarsäure, Bernsteinsäure, Benzoldicarbonsäuren, Naphthalindicarbonsäuren, Biphenyldicarbonsäuren wie beispielsweise 4,4'-Biphenyldicarbonsäure (BPDC), Pyrazindicarbonsäuren, wie 2,5-Pyrazindicarbonsäure, Bipyridindicarbonsäuren wie beispielsweise 2,2'-Bipyridindicarbonsäuren wie beispielsweise 2,2'-Bipyridin-5,5'-dicarbonsäure, Benzoltricarbonsäuren wie beispielsweise 1,2,3-, 1,2,4-Benzoltricarbonsäure oder 1,3,5-Benzoltricarbonsäure (BTC), Benzoltetracarbonsäure, Adamantantetracarbonsäure (ATC), Adamantandibenzoat (ADB) Benzoltribenzoat (BTB), Methantetrabenzoat (MTB), Adamantantetrabenzoat oder Dihydroxyterephthalsäuren wie beispielsweise 2,5-Dihydroxyterephthalsäure (DHBDC) eingesetzt.

Ganz besonders bevorzugt werden unter anderem Isophtalsäure, Terephthalsäure, 2,5-Dihydroxyterephthalsäure, 1,2,3-Benzoltricarbonsäure, 1,3,5-Benzoltricarbonsäure, 2,6-Naphthalindicarbonsäure, 1,4-Naphthalindicarbonsäure, 1,2,3,4- und 1,2,4,5-Benzoltetracarbonsäure, Campherdicarbonsäure oder 2,2'-Bipyridin-5,5'-dicarbonsäure eingesetzt.

Ein weiterer Gegenstand der vorliegenden Erfindung ist ein Verfahren zur Herstellung eines erfindungsgemäßen Gerüstmaterials den Schritt enthaltend
- Inkontaktbringen eines Metallions M mit einer gegebenenfalls deprotonierten, mindestens zweizähnigen organischen Verbindung L, die mindestens eine funktionelle Gruppe G aufweist, die nicht koordinativ an M bindet und ausgewählt ist aus der Gruppe bestehend aus -SO₃H sowie deren deprotoniertes Analogon unter Ausbildung des erfindungsgemäßen Gerüstmaterials.

In dem oben beschriebenen Verfahren ist die Gruppe G bereits in der organischen Verbindung L enthalten, so dass keine weiteren Konvertierungsschritte erforderlich sind. Hierbei muss jedoch beachtet werden, dass eine ausreichende Anzahl der Gruppe G in freier Form vorliegt und nicht an der Gerüstbildung beteiligt ist.

Ein weiterer Gegenstand der vorliegenden Erfindung ist ein Verfahren zur Herstellung eines erfindungsgemäßen Gerüstmaterials die Schritte enthaltend
- Inkontaktbringen eines Metallions M mit einer gegebenenfalls deprotonierten mindestens zweizähnigen organischen Verbindung L', die mindestens eine S-haltige Gruppe G' aufweist, die bevorzugt nicht koordinativ an M bindet und
- Umwandlung der Gruppe G' in eine Gruppe G an L.

Bei diesem Verfahren wird eine Precursorgruppe G' eingesetzt, die bereits bei der Gerüstbildung von der mindestens zweizähnigen organischen Verbindung L' mit eingeführt wird. L' leitet sich von L dadurch ab, dass diese bei Bildung der Gruppe G zu L wird. Somit unterscheidet sich L' von L zumindest in der Gruppe G. L' kann jedoch bei der Umwandlung von G' zu G weiteren chemischen Derivatisierungen ausgesetzt sein, so dass L' sich noch in weiteren chemischen Strukturmerkmalen von L unterscheiden kann. Vorzugsweise wird die Gruppe G' derart ausgewählt, dass diese nicht bei der Gerüstbildung beteiligt sein kann. Nach der Ausbildung des metallorganischen Gerüsts kann dann die Gruppe G' in die gewünschte Gruppe G umgewandelt werden. Bevorzugte Gruppen G' sind entweder Esterderivate der Gruppe G, also Sulfonsäureester oder deren Halogenide, Anhydride oder Acetale, die durch einfache Hydrolyse in die gewünschte Gruppe G umgewandelt werden können. Bevorzugt sind hierbei Sulfonsäureester.

Weiterhin kann es sich bei der Gruppe G' um Schwefelverbindungen handeln, die in einer niedrigeren Oxidationsstufe vorliegen. Hierbei ist prinzipiell jede Oxidationsstufe möglich. Eine Überführung in die Gruppe G erfolgt durch dem Fachmann bekannte Methoden der Oxidation. Beispielhafte Gruppen G' sind Thiole, Sulfide, Disulfide, Sulfite oder Sulfinate. Gängige Oxidationsmittel sind beispielsweise Peroxide, Luft, Sauerstoff, Permanganat oder Chromate.

In einer bevorzugten Ausführungsform ist G' ein Sulfonat, Sulfit-, Disulfit-, Sulfinatgruppe sowie deren Säure, Ester und Halogenid, oder Mercaptogruppe. Ein weiterer Gegenstand der vorliegenden Erfindung ist ein Verfahren zur Herstellung eines erfindungsgemäßen Gerüstmaterials, die Schritte enthaltend
- Umsetzung eines porösen metallorganischen Gerüstmaterials enthaltend mindestens eine an mindestens ein Metallion M koordinativ gebundene mindestens zweizähnige organische Verbindung L', welche einen Aromaten oder eine vinylische Doppelbindung enthält, mit einer S-haltigen Verbindung zur Bildung einer Gruppe G an L oder einer Gruppe G' an L und
- sofern G' gebunden ist, Umwandlung von G' zu G.

Bei diesem Verfahren wird die Gruppe G in ein poröses metallorganisches Gerüstmaterial eingefügt, wobei keine der mindestens zweizähnigen organischen Verbindungen L des Gerüstmaterials eine Precursorgruppe aufweist. Hierbei kann es sich bei den porösen metallorganischen Gerüstmaterialien um bekannte Gerüstmaterialien des Standes der Technik handeln. Eine Möglichkeit der Durchführung des oben beschriebenen Verfahrens ist die direkte Sulfonierung. Diese kann beispielsweise an einen Aromaten, der Teil der organischen Verbindung L' ist, stattfinden. Im Übrigen gilt für L' das oben ausgeführte. L' unterscheidet sich von L zumindest im Fehlen der Gruppe G. Vorzugsweise handelt es sich bei dem Aromaten um eine Phenyl- oder Naphthylgruppe. Es kann jedoch auch eine Sulfonierung beispielsweise an eine Doppelbindung, wie eine vinylische Doppelbindung, erfolgen. Sulfonierungsreagenzien können SO₃, H₂SO₄, Oleum, Chlorsulfonsäuren oder Sulfonylchlorid beziehungsweise Sulfurylchlorid sein. Auch hier kann gegebenenfalls die Gruppe G' durch Hydrolyse und/oder Oxidation oder auf andere Weise in G überführt werden.

Nachfolgend sind Beispiele für im Stand der Technik bekannte metallorganische Gerüstmaterialien angegeben. Neben der Kennzeichnung des MOF, dem Metall sowie dem mindestens zweizähnigen Liganden ist weiterhin das Lösemittel sowie die Zellenparameter (Winkel α, β und γ sowie die Abstände A, B und C in Å) angegeben. Letztere wurden durch Röntgenbeugung bestimmt.

| **MOF-n** | **Inhaltsstoffe molares Verhältnis M+L** | **Solvens s** | α | β | γ | **A** | **b** | **c** | **Raumgruppe** |
|---|---|---|---|---|---|---|---|---|---|
| MOF-0 | Zn(NO₃)₂·6H₂O H₃(BTC) | Ethanol | 90 | 90 | 120 | 16.711 | 16.711 | 14.189 | P6(3)/ Mcm |
| MOF-2 | Zn(NO₃)₂·6H₂O (0.246 mmol) Hz(BDC) (0.241 mmol) | DMF Toluen | 90 | 102.8 | 90 | 6.718 | 15.49 | 12.43 | P2(1)/n |
| MOF-3 | Zn(NO₃)₂·6H₂O (1.89 mmol) H₂(BDC) (1.93 mmol) | DMF MeOH | 99.72 | 111.11 | 108.4 | 9.726 | 9.911 | 10.45 | P-1 |
| MOF-4 | Zn(NO₃)₂·6H₂O (1.00 mmol) H₃(BTC) (0.5 mmol) | Ethanol | 90 | 90 | 90 | 14.728 | 14.728 | 14.728 | P2(1)3 |
| MOF-5 | Zn(NO₃)₂·6H₂O (2.22 mmol) H₂(BDC) (2.17 mmol) | DMF Chlor-benzen | 90 | 90 | 90 | 25.669 | 25.669 | 25.669 | Fm-3m |
| MOF-38 | Zn(NO₃)₂·6H₂O (0.27 mmol) H₃(BTC) (0.15 mmol) | DMF Chlor-benzen | 90 | 90 | 90 | 20.657 | 20.657 | 17.84 | I4cm |
| MOF-31 Zn(ADC)₂ | Zn(NO₃)₂·6H₂O 0.4 mmol H₂(ADC) 0.8 mmol | Ethanol | 90 | 90 | 90 | 10.821 | 10.821 | 10.821 | Pn(-3)m |
| MOF-12 Zn₂(ATC) | Zn(NO₃)₂·6H₂O 0.3 mmol H₄(ATC) 0.15 mmol | Ethanol | 90 | 90 | 90 | 15.745 | 16.907 | 18.167 | Pbca |
| MOF-20 ZnNDC | Zn(NO₃)₂·6H₂O 0.37 mmol H₂NDC 0.36 mmol | DMF Chlor-benzen | 90 | 92.13 | 90 | 8.13 | 16.444 | 12.807 | P2(1)/c |
| MOF-37 | Zn(NO₃)₂·6H₂O 0.2 mmol H₂NDC 0.2 mmol | DEF Chlor-benzen | 72.38 | 83.16 | 84.33 | 9.952 | 11.576 | 15.556 | P-1 |
| MOF-8 Tb₂ (ADC) | Tb(NO₃)₃·5H₂O 0.10 mmol H₂ADC 0.20 mmol | DMSO MeOH | 90 | 115.7 | 90 | 19.83 | 9.822 | 19.183 | C2/c |
| MOF-9 Tb₂ (ADC) | Tb(NO₃)₃·5H₂O 0.08 mmol H₂ADB 0.12 mmol | DMSO | 90 | 102.09 | 90 | 27.056 | 16.795 | 28.139 | C2/c |
| MOF-6 | Tb(NO₃)₃·5H₂O 0.30 mmol H₂ (BDC) 0.30 mmol | DMF MeOH | 90 | 91.28 | 90 | 17.599 | 19.996 | 10.545 | P21/c |
| MOF-7 | Tb(NO₃)₃·5H₂O 0.15 mmol H₂(BDC) 0.15 mmol | H₂O | 102.3 | 91.12 | 101.5 | 6.142 | 10.069 | 10.096 | P-1 |
| MOF-69A | Zn(NO₃)₂·6H₂O 0.083 mmol 4,4'BPDC 0.041 mmol | DEF H₂O₂ MeNH₂ | 90 | 111.6 | 90 | 23.12 | 20.92 | 12 | C2/c |
| MOF-69B | Zn(NO₃)₂·6H₂O 0.083 mmol 2,6-NCD 0.041 mmol | DEF H₂O₂ MeNH₂ | 90 | 95.3 | 90 | 20.17 | 18.55 | 12.16 | C2/c |
| MOF-11 Cu₂(ATC) | Cu(NO₃)₂·2.5H₂O 0.47 mmol H₂ATC 0.22 mmol | H₂O | 90 | 93.86 | 90 | 12.987 | 11.22 | 11.336 | C2/c |
| MOF-11 Cu₂(ATC) dehydr. | | | 90 | 90 | 90 | 8.4671 | 8.4671 | 14.44 | P42/ mmc |
| MOF-14 Cu₃ (BTB) | Cu(NO₃)₂·2.5H₂O 0.28 mmol H₃BTB 0.052 mmol | H₂O DMF EtOH | 90 | 90 | 90 | 26.946 | 26.946 | 26.946 | Im-3 |
| MOF-32 Cd(ATC) | Cd(NO₃)₂·4H₂O 0.24 mmol H₄ATC 0.10 mmol | H₂O NaOH | 90 | 90 | 90 | 13.468 | 13.468 | 13.468 | P(-4)3m |
| MOF-33 Zn₂ (ATB) | ZnCl₂ 0.15 mmol H₄ATB 0.02 mmol | H₂O DMF EtOH | 90 | 90 | 90 | 19.561 | 15.255 | 23.404 | Imma |
| MOF-34 Ni(ATC) | Ni(NO₃)₂·6H₂O 0.24 mmol H₄ATC 0.10 mmol | H₂O NaOH | 90 | 90 | 90 | 10.066 | 11.163 | 19.201 | P2₁2₁2₁ |
| MOF-36 Zn₂ (MTB) | Zn(NO₃)₂·4H₂O 0.20 mmol H₄MTB 0.04 mmol | H₂O DMF | 90 | 90 | 90 | 15.745 | 16.907 | 18.167 | Pbca |
| MOF-39 Zn₃O(HBTB) | Zn(NO₃)₂4H₂O 0.27 mmol H₃BTB 0.07 mmol | H₂O DMF EtOH | 90 | 90 | 90 | 17.158 | 21.591 | 25.308 | Pnma |
| N0305 | FeCl₂·4H₂O 5.03 mmol Ameisensäure. 86.90 mmol | DMF | 90 | 90 | 120 | 8.2692 | 8.2692 | 63.566 | R-3c |
| N0306A | FeCl₂·4H₂O 5.03 mmol Ameisensäure. 186.90 mmol | DEF | 90 | 90 | 90 | 9.9364 | 18.374 | 18.374 | Pbcn |
| NO29 MOF-0 ähn-lich | Mn(Ac)₂·4H₂O 0.46 mmol H₃BTC 0.69 mmol | DMF | 120 | 90 | 90 | 14.16 | 33.521 | 33.521 | P-1 |
| BPR48 A2 | Zn(NO₃)₂·6H₂O 0.012 mmol H₂BDC 0.012 mmol | DMSO Toluen | 90 | 90 | 90 | 14.5 | 17.04 | 18.02 | Pbca |
| BPR69 B1 | Cd(NO₃)₂ 4H₂O 0.0212 mmol H₂BDC 0.0428 mmol | DMSO | 90 | 98.76 | 90 | 14.16 | 15.72 | 17.66 | Cc |
| BPR92 A2 | Co(NO₃)₂·6H₂O 0.018 mmol H₂BDC 0.018 mmol | NMP | 106.3 | 107.63 | 107.2 | 7.5308 | 10.942 | 11.025 | P1 |
| BPR95 C5 | Cd(NO₃)₂ 4H₂O 0.012 mmol H₂BDC 0.36 mmol | NMP | 90 | 112.8 | 90 | 14.460 | 11.085 | 15.829 | P2(1)/n |
| Cu C₆H₄O₆ | Cu(NO₃)₂·2.5H₂O 0.370 mmol H₂BDC(OH)₂ 0.37 mmol | DMF Chlor-benzen | 90 | 105.29 | 90 | 15.259 | 14.816 | 14.13 | P2(1)/c |
| M(BTC) MOF-0 ähnlich | Co(SO₄) H₂O 0.055 mmol H₃BTC 0.037 mmol | DMF | wie MOF-0 | | | | | | |
| Tb(C₆H₄O₆) | Tb(NO₃)₃·5H₂O 0.370 mmol H₂(C₆H₄O₆) 0.56 mmol | DMF Chlor-benzen | 104.6 | 107.9 | 97.147 | 10.491 | 10.981 | 12.541 | P-1 |
| Zn (C₂O₄) | ZnCl₂ 0.370 mmol Oxalsäure 0.37 mmol | DMF Chlor-benzen | 90 | 120 | 90 | 9.4168 | 9.4168 | 8.464 | P(-3)1m |
| Co(CHO) | Co(NO₃)₂·5H₂O 0.043 mmol Ameisensäure 1.60 mmol | DMF | 90 | 91.32 | 90 | 11.328 | 10.049 | 14.854 | P2(1)/n |
| Cd(CHO) | Cd(NO₃)₂·4H₂O 0.185 mmol formic acid 0.185 mmol | DMF | 90 | 120 | 90 | 8.5168 | 8.5168 | 22.674 | R-3c |
| Cu(C₃H₂O₄) | Cu(NO₃)₂·2.5H₂O 0.043 mmol Malonsäure. 0.192 mmol | DMF | 90 | 90 | 90 | 8.366 | 8.366 | 11.919 | P43 |
| Zn₆ (NDC)₅ | Zn(NO₃)₂·6H₂O | DMF | 90 | 95.902 | 90 | 19.504 | 16.482 | 14.64 | C2/m |
| MOF-48 | 0.097 mmol 14 NDC 0.069 mmol | Chlorbenzen H₂O₂ | | | | | | | |
| MOF-47 | Zn(NO₃)₂6H₂O 0.185 mmol H₂(BDC[CH₃]₄) 0.185 mmol | DMF Chlor-benzen H₂O₂ | 90 | 92.55 | 90 | 11.303 | 16.029 | 17.535 | P2(1)/c |
| M025 | Cu(NO₃)₂·2.5H₂O 0.084 mmol BPhDC 0.085 mmol | DMF | 90 | 112.0 | 90 | 23.880 | 16.834 | 18.389 | P2(1)/c |
| Cu-Thio | Cu(NO₃)₂·2.5H₂O 0.084 mmol Thiophen Dicarbonsäure 0.085 mmol | DEF | 90 | 113.6 | 90 | 15.4747 | 14.514 | 14.032 | P2(1)/c |
| CIBDC1 | Cu(NO₃)₂·2.5H₂O 0. 084 mmol H₂(BDCCl₂) 0.085 mmol | DMF | 90 | 105.6 | 90 | 14.911 | 15.622 | 18.413 | C2/c |
| MOF-101 | Cu(NO₃)₂·2.5H₂O 0.084 mmol BrBDC 0.085 mmol | DMF | 90 | 90 | 90 | 21.607 | 20.607 | 20.073 | Fm3m |
| Zn₃(BTC)₂ | ZnCl₂ 0.033 mmol H₃BTC 0.033 mmol | DMF EtOH Base zugegeben | 90 | 90 | 90 | 26.572 | 26.572 | 26.572 | Fm-3m |
| MOF-j | Co(CH₃CO₂)₂·4H₂O (1.65 mmol) H₃(BZC) (0.95 mmol) | H₂O | 90 | 112.0 | 90 | 17.482 | 12.963 | 6.559 | C2 |
| MOF-n | Zn(NO₃)₂·6H₂O H₃ (BTC) | Ethanol | 90 | 90 | 120 | 16.711 | 16.711 | 14.189 | P6(3)/mcm |
| PbBDC | Pb(NO₃)₂ (0.181 mmol) H₂(BDC) (0.181 mmol) | DMF Ethanol | 90 | 102.7 | 90 | 8.3639 | 17.991 | 9.9617 | P2(1)/n |
| Znhex | Zn(NO₃)₂·6H₂O (0.171 mmol) H₃BTB (0.114 mmol) | DMF p-Xylen Ethanol | 90 | 90 | 120 | 37.1165 | 37.117 | 30.019 | P3(1)c |
| AS16 | FeBr₂ 0.927 mmol H₂(BDC) 0.927 mmol | DMF anhydr. | 90 | 90.13 | 90 | 7.2595 | 8.7894 | 19.484 | P2(1)c |
| AS27-2 | FeBr₂ 0.927 mmol H₃(BDC) 0.464 mmol | DMF anhydr. | 90 | 90 | 90 | 26.735 | 26.735 | 26.735 | Fm3m |
| AS32 | FeCl₃ 1.23 mmol H₂(BDC) 1.23 mmol | DMF anhydr. Ethanol | 90 | 90 | 120 | 12.535 | 12.535 | 18.479 | P6(2)c |
| AS54-3 | FeBr₂ 0.927 BPDC 0.927 mmol | DMF anhydr. n-Propanol | 90 | 109.98 | 90 | 12.019 | 15.286 | 14.399 | C2 |
| AS61-4 | FeBr₂ 0.927 mmol m-BDC 0.927 mmol | Pyridin anhydr. | 90 | 90 | 120 | 13.017 | 13.017 | 14.896 | P6(2)c |
| AS68-7 | FeBr₂ 0.927 mmol m-BDC 1.204 mmol | DMF anhydr. Pyridin | 90 | 90 | 90 | 18.3407 | 10.036 | 18.039 | Pca2₁ |
| Zn(ADC) | Zn(NO₃)₂·6H₂O 0.37 mmol H₂(ADC) 0.36 mmol | DMF Chlor-benzen | 90 | 99.85 | 90 | 16.764 | 9.349 | 9.635 | C2/c |
| MOF-12 Zn₂ (ATC) | Zn(NO₃)₂·6H₂O 0.30 mmol H₄(ATC) 0.15 mmol | Ethanol | 90 | 90 | 90 | 15.745 | 16.907 | 18.167 | Pbca |
| MOF-20 ZnNDC | Zn(NO₃)₂·6H₂O 0.37 mmol H₂NDC 0.36 mmol | DMF Chlor-benzen | 90 | 92.13 | 90 | 8.13 | 16.444 | 12.807 | P2(1)/c |
| MOF-37 | Zn(NO₃)₂·6H₂O 0.20 mmol H₂NDC 0.20 mmol | DEF Chlor-benzen | 72.38 | 83.16 | 84.33 | 9.952 | 11.576 | 15.556 | P-1 |
| Zn(NDC) (DMSO) | Zn(NO₃)₂·6H₂O H₂NDC | DMSO | 68.08 | 75.33 | 88.31 | 8.631 | 10.207 | 13.114 | P-1 |
| Zn(NDC) | Zn(NO₃)₂·6H₂O H₂NDC | | 90 | 99.2 | 90 | 19.289 | 17.628 | 15.052 | C2/c |
| Zn(HPDC) | Zn(NO₃)₂·4H₂O 0.23 mmol H₂(HPDC) 0.05 mmol | DMF H₂O | 107.9 | 105.06 | 94.4 | 8.326 | 12.085 | 13.767 | P-1 |
| Co(HPDC) | Co(NO₃)₂·6H₂O 0.21 mmol H₂ (HPDC) 0.06 mmol | DMF H₂O/ Ethanol | 90 | 97.69 | 90 | 29.677 | 9.63 | 7.981 | C2/c |
| Zn₃(PDC) 2.5 | Zn(NO₃)₂·4H₂O 0.17 mmol H₂(HPDC) 0.05 mmol | DMF/ CIBz H₂0/ TEA | 79.34 | 80.8 | 85.83 | 8.564 | 14.046 | 26.428 | P-1 |
| Cd₂ (TPDC)2 | Cd(NO₃)₂·4H₂O 0.06 mmol H₂(HPDC) 0.06 mmol | Methanol/ CHP H₂O | 70.59 | 72.75 | 87.14 | 10.102 | 14.412 | 14.964 | P-1 |
| Tb(PDC)1. 5 | Tb(NO₃)₃·5H₂O 0.21 mmol H₂(PDC) 0.034 mmol | DMF H₂O/ Ethanol | 109.8 | 103.61 | 100.14 | 9.829 | 12.11 | 14.628 | P-1 |
| ZnDBP | Zn(NO₃)₂·6H₂O 0.05 mmol Dibenzylphosphat 0.10 mmol | MeOH | 90 | 93.67 | 90 | 9.254 | 10.762 | 27.93 | P2/n |
| Zn₃(BPDC) | ZnBr₂ 0.021 mmol 4,4'BPDC 0.005 mmol | DMF | 90 | 102.76 | 90 | 11.49 | 14.79 | 19.18 | P21/n |
| CdBDC | Cd(NO₃)₂·4H₂O 0.100 mmol H₂(BDC) 0.401 mmol | DMF Na₂SiO ₃ (aq) | 90 | 95.85 | 90 | 11.2 | 11.11 | 16.71 | P21/n |
| Cd-mBDC | Cd(NO₃)₂·4H₂O 0.009 mmol H₂(mBDC) 0.018 mmol | DMF MeNH₂ | 90 | 101.1 | 90 | 13.69 | 18.25 | 14.91 | C2/c |
| Zn₄OBND C | Zn(NO₃)₂·6H₂O 0.041 mmol BNDC | DEF MeNH₂ H₂O₂ | 90 | 90 | 90 | 22.35 | 26.05 | 59.56 | Fmmm |
| Eu(TCA) | Eu(NO₃)₃·6H₂O 0.14 mmol TCA 0.026 mmol | DMF Chlorbenzen | 90 | 90 | 90 | 23.325 | 23.325 | 23.325 | Pm-3n |
| Tb(TCA) | Tb(NO₃)₃·6H₂O 0.069 mmol TCA 0.026 mmol | DMF Chlorbenzen | 90 | 90 | 90 | 23.272 | 23.272 | 23.372 | Pm-3n |
| Formate | Ce(NO₃)₃·6H₂O 0.138 mmol Ameisensäure. 0.43 mmol | H₂O Ethanol | 90 | 90 | 120 | 10.668 | 10.667 | 4.107 | R-3m |
| | FeCl₂·4H₂O 5.03 mmol Ameisensre. 86.90 mmol | DMF | 90 | 90 | 120 | 8.2692 | 8.2692 | 63.566 | R-3c |
| | FeCl₂·4H₂O 5.03 mmol Ameisensäure. 86.90 mmol | DEF | 90 | 90 | 90 | 9.9364 | 18.374 | 18.374 | Pbcn |
| | FeCl₂·4H₂O 5.03 mmol Ameisensäure. 86.90 mmol | DEF | 90 | 90 | 90 | 8.335 | 8.335 | 13.34 | P-31c |
| N0330 | FeCl₂·4H₂O 0.50 mmol Ameisensre. 8.69 mmol | Formamid | 90 | 90 | 90 | 8.7749 | 11.655 | 8.3297 | Pnna |
| NO332 | FeCl₂·4H₂O 0.50 mmol Ameisensäure. 8.69 mmol | DIP | 90 | 90 | 90 | 10.0313 | 18.808 | 18.355 | Pbcn |
| NO333 | FeCl₂·4H₂O 0.50 mmol Ameisensäure. 8.69 mmol | DBF | 90 | 90 | 90 | 45.2754 | 23.861 | 12.441 | Cmcm |
| NO335 | FeCl₂·4H₂O 0.50 mmol Ameisensäure. 8.69 mmol | CHF | 90 | 91.372 | 90 | 11.5964 | 10.187 | 14.945 | P21/n |
| NO336 | FeCl₂·4H₂O 0.50 mmol Ameisensäure. 8.69 mmol | MFA | 90 | 90 | 90 | 11.7945 | 48.843 | 8.4136 | Pbcm |
| NO13 | Mn(Ac)₂·4H₂O 0.46 mmol Benzoesäure. 0.92 mmol Bipyridin 0.46 mmol | Ethanol | 90 | 90 | 90 | 18.66 | 11.762 | 9.418 | Pbcn |
| N029 MOF-0 ähnlich | Mn(Ac)₂·4H₂O 0.46 mmol H₃BTC 0.69 mmol | DMF | 120 | 90 | 90 | 14.16 | 33.521 | 33.521 | P-1 |
| Mn(hfac)₂ (O₂CC₆H₅) | Mn(Ac)₂·4H₂O 0.46 mmol Hfac 0.92 mmol Bipyridin 0.46 mmol | Ether | 90 | 95.32 | 90 | 9.572 | 17.162 | 14.041 | C2/c |
| BPR43G2 | Zn(NO₃)₂·6H₂O 0.0288 mmol H₂BDC 0.0072 mmol | DMF CH₃CN | 90 | 91.37 | 90 | 17.96 | 6.38 | 7.19 | C2/c |
| BPR48A2 | Zn(NO₃)₂ 6H₂O 0.012 mmol H₂BDC 0.012 mmol | DMSO Toluen | 90 | 90 | 90 | 14.5 | 17.04 | 18.02 | Pbca |
| BPR49B1 | Zn(NO₃)₂ 6H₂O 0.024 mmol H₂BDC 0.048 mmol | DMSO Metha-nol | 90 | 91.172 | 90 | 33.181 | 9.824 | 17.884 | C2/c |
| BPR56E1 | Zn(NO₃)₂ 6H₂O 0.012 mmol H₂BDC 0.024 mmol | DMSO n-Propanol | 90 | 90.096 | 90 | 14.5873 | 14.153 | 17.183 | P2(1)/n |
| BPR68D10 | Zn(NO₃)₂ 6H₂O 0.0016 mmol H₃BTC 0.0064 mmol | DMSO Benzen | 90 | 95.316 | 90 | 10.0627 | 10.17 | 16.413 | P2(1)/c |
| BPR69B1 | Cd(NO₃)₂ 4H₂O 0.0212 mmol H₂BDC 0.0428 mmol | DMSO | 90 | 98.76 | 90 | 14.16 | 15.72 | 17.66 | Cc |
| BPR73E4 | Cd(NO₃)₂ 4H₂O 0.006 mmol H₂BDC 0.003 mmol | DMSO Toluen | 90 | 92.324 | 90 | 8.7231 | 7.0568 | 18.438 | P2(1)/n |
| BPR76D5 | Zn(NO₃)₂6H₂O 0.0009 mmol H₂BzPDC 0.0036 mmol | DMSO | 90 | 104.17 | 90 | 14.4191 | 6.2599 | 7.0611 | Pc |
| BPR80B5 | Cd(NO₃)₂·4H₂O 0.018 mmol H₂BDC 0.036 mmol | DMF | 90 | 115.11 | 90 | 28.049 | 9.184 | 17.837 | C2/c |
| BPR80H5 | Cd(NO₃)₂ 4H₂O 0.027 mmol H₂BDC 0.027 mmol | DMF | 90 | 119.06 | 90 | 11.4746 | 6.2151 | 17.268 | P2/c |
| BPR82C6 | Cd(NO₃)₂ 4H₂O 0.0068 mmol H₂BDC 0.202 mmol | DMF | 90 | 90 | 90 | 9.7721 | 21.142 | 27.77 | Fdd2 |
| BPR86C3 | Co(NO₃)₂6H₂O 0.0025 mmol H₂BDC 0.075 mmol | DMF | 90 | 90 | 90 | 18.3449 | 10.031 | 17.983 | Pca2(1) |
| BPR86H6 | Cd(NO₃)₂6H₂O 0.010 mmol H₂BDC 0.010 mmol | DMF | 80.98 | 89.69 | 83.412 | 9.8752 | 10.263 | 15.362 | P-1 |
| | Co(NO₃)₂ 6H₂O | NMP | 106.3 | 107.63 | 107.2 | 7.5308 | 10.942 | 11.025 | P1 |
| BPR95A2 | Zn(NO₃)₂6H₂O 0.012 mmol H₂BDC 0.012 mmol | NMP | 90 | 102.9 | 90 | 7.4502 | 13.767 | 12.713 | P2(1)/c |
| CuC₆F₄O₄ | Cu(NO₃)₂·2.5H₂O 0.370 mmol H₂BDC(OH)₂ 0.37 mmol | DMF Chlor-benzen | 90 | 98.834 | 90 | 10.9675 | 24.43 | 22.553 | P2(1)/n |
| Fe Formic | FeCl₂·4H₂O 0.370 mmol Ameisensäure. 0.37 mmol | DMF | 90 | 91.543, | 90 | 11.495 | 9.963 | 14.48 | P2(1)/n |
| Mg Formic | Mg(NO₃)₂·6H₂O 0.370 mmol Ameisensäure. 0.37 mmol | DMF | 90 | 91.359 | 90 | 11.383 | 9.932 | 14.656 | P2(1)/n |
| MgC₆H₄O₆ | Mg(NO₃)₂·6H₂O 0.370 mmol H₂BDC(OH)₂ 0.37 mmol | DMF | 90 | 96.624 | 90 | 17.245 | 9.943 | 9.273 | C2/c |
| Zn C₂H₄BDC MOF-38 | ZnCl₂ 0.44 mmol CBBDC 0.261 mmol | DMF | 90 | 94.714 | 90 | 7.3386 | 16.834 | 12.52 | P2(1)/n |
| MOF-49 | ZnCl₂ 0.44 mmol m-BDC 0.261 mmol | DMF CH₃CN | 90 | 93.459 | 90 | 13.509 | 11.984 | 27.039 | P2/c |
| MOF-26 | Cu(NO₃)₂·5H₂O 0.084 mmol DCPE 0.085 mmol | DMF | 90 | 95.607 | 90 | 20.8797 | 16.017 | 26.176 | P2(1)/n |
| MOF-112 | Cu(NO₃)_{2·}2.5H₂O 0.084 mmol o-Br-m-BDC 0.085 mmol | DMF Ethanol | 90 | 107.49 | 90 | 29.3241 | 21.297 | 18.069 | C2/c |
| MOF-109 | Cu(NO₃)_{2·}2.5H₂O 0.084 mmol KDB 0.085 mmol | DMF | 90 | 111.98 | 90 | 23.8801 | 16.834 | 18.389 | P2(1)/c |
| MOF-111 | Cu(NO₃)_{2·}2.5H₂O 0.084 mmol o-BrBDC 0.085 mmol | DMF Ethanol | 90 | 102.16 | 90 | 10.6767 | 18.781 | 21.052 | C2/c |
| MOF-110 | Cu(NO₃)₂·2.5H₂O 0.084 mmol Thiophen Dicarbonsäure 0.085 mmol | DMF | 90 | 90 | 120 | 20.0652 | 20.065 | 20.747 | R-3/m |
| MOF-107 | Cu(NO₃)₂·2.5H₂O 0.084 mmol Thiophen Dicarbonsäure 0.085 mmol | DEF | 104.8 | 97.075 | 95.206 | 11.032 | 18.067 | 18.452 | P-1 |
| MOF-108 | Cu(NO₃)₂·2.5H₂O 0.084 mmol Thiophen Dicarbonsäure 0.085 mmol | DBF/ Methanol | 90 | 113.63 | 90 | 15.4747 | 14.514 | 14.032 | C2/c |
| MOF-102 | Cu(NO₃)₂·2.5H₂O 0.084 mmol H₂(BDCCl₂) 0.085 mmol | DMF | 91.63 | 106.24 | 112.01 | 9.3845 | 10.794 | 10.831 | P-1 |
| Clbdc1 | Cu(NO₃)₂·2.5H₂O 0.084 mmol H₂(BDCCl₂) 0.085 mmol | DEF | 90 | 105.56 | 90 | 14.911 | 15.622 | 18.413 | P-1 |
| Cu(NMOP) | Cu(NO₃)₂·2.5H₂O 0.084 mmol NBDC 0.085 mmol | DMF | 90 | 102.37 | 90 | 14.9238 | 18.727 | 15.529 | P2(1)/m |
| Tb(BTC) | Tb(NO₃)₃·5HzO 0.033 mmol H₃BTC 0.033 mmol | DMF | 90 | 106.02 | 90 | 18.6986 | 11.368 | 19.721 | |
| Zn₃(BTC)₂ Honk | ZnCl₂ 0.033 mmol H₃BTC 0.033 mmol | DMF Ethanol | 90 | 90 | 90 | 26.572 | 26.572 | 26.572 | Fm-3m |
| Zn₄O(NDC) | Zn(NO₃)₂·4H₂O 0.066 mmol 14NDC 0.066 mmol | DMF Ethanol | 90 | 90 | 90 | 41.5594 | 18.818 | 17.574 | aba2 |
| CdTDC | Cd(NO₃)₂·4H₂O 0.014 mmol Thiophen 0.040 mmol DABCO 0.020 mmol | DMF H₂O | 90 | 90 | 90 | 12.173 | 10.485 | 7.33 | Pmma |
| IRMOF-2 | Zn(NO₃)₂·4H₂O 0.160 mmol o-Br-BDC 0.60 mmol | DEF | 90 | 90 | 90 | 25.772 | 25.772 | 25.772 | Fm-3m |
| IRMOF-3 | Zn(NO₃)₂·4H₂O 0.20 mmol H₂N-BDC 0.60 mmol | DEF Ethanol | 90 | 90 | 90 | 25.747 | 25.747 | 25.747 | Fm-3m |
| IRMOF-4 | Zn(NO₃)₂·4H₂O 0.11 mmol [C₃H₇O]₂-BDC 0.48 mmol | DEF | 90 | 90 | 90 | 25.849 | 25.849 | 25.849 | Fm-3m |
| IRMOF-5 | Zn(NO₃)₂·4H₂O 0.13 mmol [C5H₁₁O]₂-BDC 0.50 mmol | DEF | 90 | 90 | 90 | 12.882 | 12.882 | 12.882 | Pm-3m |
| IRMOF-6 | Zn(NO₃)₂·4H₂O 0.20 mmol [C₂H₄]-BDC 0.60 mmol | DEF | 90 | 90 | 90 | 25.842 | 25.842 | 25.842 | Fm-3m |
| IRMOF-7 | Zn(NO₃)₂·4H₂O 0.07 mmol 1,4NDC 0.20 mmol | DEF | 90 | 90 | 90 | 12.914 | 12.914 | 12.914 | Pm-3m |
| IRMOF-8 | Zn(NO₃)₂·4H₂O 0.55 mmol 2,6NDC 0.42 mmol | DEF | 90 | 90 | 90 | 30.092 | 30.092 | 30.092 | Fm-3m |
| IRMOF-9 | Zn(NO₃)₂·4H₂O 0.05 mmol BPDC 0.42 mmol | DEF | 90 | 90 | 90 | 17.147 | 23.322 | 25.255 | Pnnm |
| IRMOF-10 | Zn(NO₃)₂·4H₂O 0.02 mmol BPDC 0.012 mmol | DEF | 90 | 90 | 90 | 34.281 | 34.281 | 34.281 | Fm-3m |
| IRMOF-11 | Zn(NO₃)₂·4H₂O 0.05 mmol HPDC 0.20 mmol | DEF | 90 | 90 | 90 | 24.822 | 24.822 | 56.734 | R-3m |
| IRMOF-12 | Zn(NO₃)₂·4H₂O 0.017 mmol HPDC 0.12 mmol | DEF | 90 | 90 | 90 | 34.281 | 34.281 | 34.281 | Fm-3m |
| IRMOF-13 | Zn(NO₃)₂·4H₂O 0.048 mmol PDC 0.31 mmol | DEF | 90 | 90 | 90 | 24.822 | 24.822 | 56.734 | R-3m |
| IRMOF-14 | Zn(NO₃)₂·4H₂O 0.17 mmol PDC 0.12 mmol | DEF | 90 | 90 | 90 | 34.381 | 34.381 | 34.381 | Fm-3m |
| IRMOF-15 | Zn(NO₃)₂·4H₂O 0.063 mmol TPDC 0.025 mmol | DEF | 90 | 90 | 90 | 21.459 | 21.459 | 21.459 | Im-3m |
| IRMOF-16 | Zn(NO₃)₂·4H₂O 0.0126 mmol TPDC 0.05 mmol | DEF NMP | 90 | 90 | 90 | 21.49 | 21.49 | 21.49 | Pm-3m |

- ADC: Acetylendicarbonsäure
- NDC: Naphthalindicarbonsäure
- BDC: Benzoldicarbonsäure
- ATC: Adamantantetracarbonsäure
- BTC: Benzoltricarbonsäure
- BTB: Benzoltribenzoesäure
- MTB: Methantetrabenzoesäure
- ATB: Adamantantetrabenzoesäure
- ADB: Adamantandibenzoesäure

Weitere MOF sind MOF-177, MOF-178, MOF-74, MOF-235, MOF-236, MOF-69 bis 80, MOF-501, MOF-502, welche in der Literatur beschrieben sind.

Die metallorganischen Gerüstmaterialien gemäß der vorliegenden Erfindung enthalten Poren, insbesondere Mikro- und/oder Mesoporen. Mikroporen sind definiert als solche mit einem Durchmesser von 2 nm oder kleiner und Mesoporen sind definiert durch einen Durchmesser im Bereich von 2 bis 50 nm, jeweils entsprechend nach der Definition, wie sie in Pure & Applied Chem. 57, (1985), Seite 603-619, insbesondere auf Seite 606 angegeben ist. Die Anwesenheit von Mikro- und/oder Mesoporen kann mit Hilfe von Sorptionsmessungen überprüft werden, wobei diese Messungen die Aufnahmekapazität der MOF für Stickstoff bei 77 Kelvin gemäß DIN 66131 und/oder DIN 66134 bestimmt.

Vorzugsweise beträgt die spezifische Oberfläche - berechnet nach dem Langmuir-Modell gemäß DIN 66135 (DIN 66131, 66134) für ein metallorganisches Gerüstmaterial in Pulverform mehr als 5 m²/g, mehr bevorzugt über 10 m²/g, mehr bevorzugt mehr als 50 m²/g, weiter mehr bevorzugt mehr als 500 m²/g, weiter mehr bevorzugt mehr als 1000 m²/g und besonders bevorzugt mehr als 1250 m²/g.

MOF Formkörper können eine niedrigere spezifische Oberfläche besitzen; vorzugsweise jedoch mehr als 10 m²/g, mehr bevorzugt mehr als 50 m²/g, weiter mehr bevorzugt mehr als 500 m²/g.

Die Porengröße des metallorganischen Gerüstmaterials kann durch Wahl des geeigneten Liganden und/oder der mindestens zweizähnigen organischen Verbindung gesteuert werden. Häufig gilt, dass je größer die organische Verbindung desto größer die Porengröße ist. Vorzugsweise beträgt die Porengröße von 0,2 nm bis 30 nm, besonders bevorzugt liegt die Porengröße im Bereich von 0,3 nm bis 9 nm bezogen auf das kristalline Material.

In einem MOF-Formkörper treten jedoch auch größere Poren auf, deren Größenverteilung variieren kann. Vorzugsweise wird jedoch mehr als 50 % des gesamten Porenvolumens, insbesondere mehr als 75 %, von Poren mit einem Porendurchmesser von bis zu 1000 nm gebildet. Vorzugsweise wird jedoch ein Großteil des Porenvolumens von Poren aus zwei Durchmesserbereichen gebildet. Es ist daher weiter bevorzugt, wenn mehr als 25 % des gesamten Porenvolumens, insbesondere mehr als 50 % des gesamten Porenvolumens von Poren gebildet wird, die in einem Durchmesserbereich von 100 nm bis 800 nm liegen und wenn mehr als 15 % des gesamten Porenvolumens, insbesondere mehr als 25 % des gesamten Porenvolumens von Poren gebildet wird, die in einem Durchmesserbereich bis zu 10 nm liegen. Die Porenverteilung kann mittels Quecksilber-Porosimetrie bestimmt werden.

Das metallorganische Gerüstmaterial kann pulverförmig beziehungsweise als Agglomerate vorliegen. Das Gerüstmaterial kann als solches verwendet werden oder es wird in einen Formkörper umgewandelt. Bevorzugte Verfahren sind hierbei die Verstrangung oder Tablettierung. Bei der Formkörperherstellung kann das Gerüstmaterial weitere Materialien, wie beispielsweise Binder, Gleitmittel oder andere Additive aufweisen, welche während der Herstellung hinzugesetzt werden. Ebenso ist es denkbar, dass das Gerüstmaterial weitere Bestandteile aufweist, wie zum Beispiel Adsorbentien wie Aktivkohle oder dergleichen.

Hinsichtlich der möglichen Geometrien der Formkörper existieren im Wesentlichen keine Beschränkungen. Beispielsweise sind unter anderem Pellets wie beispielsweise scheibenförmige Pellets, Pillen, Kugeln, Granulat, Extrudate wie beispielsweise Stränge, Waben, Gitter oder Hohlkörper zu nennen.

Zur Herstellung dieser Formkörper sind grundsätzlich sämtliche geeigneten Verfahren möglich. Es sind insbesondere folgende Verfahrensführungen bevorzugt:
- Kneten/Kollern des Gerüstmaterials allein oder zusammen mit mindestens einem Bindemittel und/oder mindestens einem Anteigungsmittel und/oder mindestens einer Templatverbindung unter Erhalt eines Gemisches; Verformen des erhaltenen Gemisches mittels mindestens einer geeigneten Methode wie beispielsweise Extrudieren; Optional Waschen und/oder Trocknen und/oder Calcinieren des Extrudates; Optional Konfektionieren.
- Aufbringen des Gerüstmaterials auf mindestens ein gegebenenfalls poröses Trägermaterial. Das erhaltene Material kann dann gemäß der vorstehend beschriebenen Methode zu einem Formkörper weiterverarbeitet werden.
- Aufbringen des Gerüstmaterials auf mindestens ein gegebenenfalls poröses Substrat.

Kneten/Kollern und Verformen kann gemäß eines jeden geeigneten Verfahrens erfolgen, wie beispielsweise in Ullmanns Enzyklopädie der Technischen Chemie, 4. Auflage, Band 2, S. 313 ff. (1972) beschrieben.

Beispielsweise kann das Kneten/Kollern und/oder Verformen mittels einer Kolbenpresse, Walzenpresse in Anwesenheit oder Abwesenheit mindestens eines Bindermaterials, Compoundieren, Pelletieren, Tablettieren, Extrudieren, Co-Extrudieren, Verschäumen, Verspinnen, Beschichten, Granulieren, bevorzugt Sprühgranulieren, Versprühen, Sprühtrocknen oder einer Kombination aus zwei oder mehr dieser Methoden erfolgen.

Ganz besonders werden Pellets und/oder Tabletten hergestellt.

Das Kneten und/oder Verformen kann bei erhöhten Temperaturen wie beispielsweise im Bereich von Raumtemperatur bis 300°C und/oder bei erhöhtem Druck wie beispielsweise im Bereich von Normaldruck bis hin zu einigen hundert bar und/oder in einer Schutzgasatmosphäre wie beispielsweise in Anwesenheit mindestens eines Edelgases, Stickstoff oder einem Gemisch aus zwei oder mehr davon erfolgen.

Das Kneten und/oder Verformen wird gemäß einer weiteren Ausführungsform unter Zugabe mindestens eines Bindemittels durchgeführt, wobei als Bindemittel grundsätzlich jede chemische Verbindung eingesetzt werden kann, die die zum Kneten und/oder Verformen gewünschte Viskosität der zu verknetenden und/oder verformenden Masse gewährleistet. Demgemäß können Bindemittel im Sinne der vorliegenden Erfindung sowohl viskositätserhöhende als auch viskositätserniedrigende Verbindungen sein.

Als unter anderem bevorzugte Bindemittel sind beispielsweise Aluminiumoxid oder Aluminiumoxid enthaltende Binder, wie sie beispielsweise in der WO 94/29408 beschrieben sind, Siliciumdioxid, wie es beispielsweise in der EP 0 592 050 A1 beschrieben ist, Mischungen ais Siliciumdioxid und Aluminiumoxid, wie sie beispielsweise in der WO 94/13584 beschrieben sind, Tonminerale, wie sie beispielsweise in der JP 03-037156 A beschrieben sind, beispielsweise Montmorillonit, Kaolin, Bentonit, Hallosit, Dickit, Nacrit und Anauxit, Alkoxysilane, wie sie beispielsweise in der EP 0 102 544 B1 beschrieben sind, beispielsweise Tetraalkoxysilane wie beispielsweise Tetramethoxysilan, Tetraethoxysilan, Tetrapropoxysilan, Tetrabutoxysilan, oder beispielsweise Trialkoxysilane wie beispielsweise Trimethoxysilan, Triethoxysilan, Tripropoxysilan, Tributoxysilan, Alkoxytitanate, beispielsweise Tetraalkoxytitanate wie beispielsweise Tetramethoxytitanat, Tetraethoxytitanat, Tetrapropoxytitanat, Tetrabutoxytitanat, oder beispielsweise Trialkoxytitanate wie beispielsweise Trimethoxytitanat, Triethoxytitanat, Tripropoxytitanat, Tributoxytitanat, Alkoxyzirkonate, beispielsweise Tetraalkoxyzirkonate wie beispielsweise Tetramethoxyzirkonat, Tetraethoxyzirkonat, Tetrapropoxyzirkonat, Tetrabutoxyzirkonat, oder beispielsweise Trialkoxyzirkonate wie beispielsweise Trimethoxyzirkonat, Triethoxyzirkonat, Tripropoxyzirkonat, Tributoxyzirkonat, Silikasole und/oder amphiphile Substanzen nennen.

Als viskositätssteigernde Verbindung kann beispielsweise auch, gegebenenfalls zusätzlich zu den oben genannten Verbindungen, eine organische Verbindung und/oder ein hydrophiles Polymer wie beispielsweise Cellulose oder ein Cellulosederivat wie beispielsweise Methylcellulose und/oder ein Polyacrylat und/oder ein Polymethacrylat und/oder ein Polyvinylalkohol und/oder ein Polyvinylpyrrolidon und/oder ein Polyisobuten und/oder ein Polytetrahydrofuran und/oder ein Polyethylenoxid eingesetzt werden.

Als Anteigungsmittel kann unter anderem bevorzugt Wasser oder mindestens ein Alkohol wie beispielsweise ein Monoalkohol mit 1 bis 4 C-Atomen wie beispielsweise Methanol, Ethanol, n-Propanol, iso-Propanol, 1-Butanol, 2-Butanol, 2-Methyl-1-propanol oder 2-Methyl-2-propanol oder ein Gemisch aus Wasser und mindestens einem der genannten Alkohole oder ein mehrwertiger Alkohol wie beispielsweise ein Glykol, bevorzugt ein wassermischbarer mehrwertiger Alkohol, allein oder als Gemisch mit Wasser und/oder mindestens einem der genannten einwertigen Alkohole eingesetzt werden.

Weitere Additive, die zum Kneten und/oder Verformen eingesetzt werden können, sind unter anderem Schmiermittel wie Graphite, Amine oder Aminderivate wie beispielsweise Tetraalkylammonium-Verbindungen oder Aminoalkohole und Carbonat enthaltende Verbindungen wie etwa Calciumcarbonat. Solche weiteren Additive sind etwa in der EP 0 389 041 A1, der EP 0 200 260 A1 oder der WO 95/19222 beschrieben.

Die Reihenfolge der Additive wie Templatverbindung, Binder, Anteigungsmittel, viskositätssteigernde Substanz beim Verformen und Kneten ist grundsätzlich nicht kritisch.

Gemäß einer weiteren bevorzugten Ausführungsform wird der gemäß Kneten und/oder Verformen erhaltene Formkörper mindestens einer Trocknung unterzogen, die im Allgemeinen bei einer Temperatur im Bereich von 25 bis 300°C, bevorzugt im Bereich von 50 bis 300°C und besonders bevorzugt im Bereich von 100 bis 300°C durchgeführt wird. Ebenso ist es möglich, im Vakuum oder unter Schutzgasatmosphäre oder durch Sprühtrocknung zu trocknen.

Gemäß einer besonders bevorzugten Ausführungsform wird im Rahmen dieses Trocknungsvorgangs mindestens eine der als Additive zugesetzten Verbindungen zumindest teilweise aus dem Formkörper entfernt.

Ein weiterer Gegenstand der vorliegenden Erfindung ist die Verwendung eines erfindungsgemäßen porösen metallorganischen Gerüstmaterials als Ionenaustauscher oder Brönstedt-Säure. Die porösen Gerüstmaterialien können beispielsweise bei chemischen Reaktionen wie Veresterungen, Veretherungen, Umesterungen, Umetherungen, Alkylierungen, Acylierungen, Isomerisierungen, De- und Hydratisierungen, Alkoxylierungen, Di-, Oligo- und Polymerisationen sowie Aminierungen eingesetzt werden.

### Beispiele

**Beispiel 1** Herstellung eines Aluminium-metallorganischen Gerüstmaterials ("Al-MOF")

250,1 g Terephthalsäure (BDC) und 292,9 g Al₂(SO₄)₃ • 18H₂O werden in 1.257 g N,N-dimethylformamid (DMF) suspendiert und unter Rühren für 24 Stunden auf 130°C erwärmt. Anschließend wird die Suspension filtriert und das Filtrat mit DMF nachgewaschen. Der Filterkuchen wird 2 Stunden bei 120°C im Trockenschrank getrocknet. Anschließend wird bei 320°C im Muffelofen während 2 Stunden calciniert.

### Beispiel 2 Herstellung eines erfindungsgemäßen sulfonierten Al-MOF

3,0 g des Al-MOF-Pulvers aus Beispiel 1 werden in einem Austauschrohr aus Glas mit P3-Glasfritte eingebracht und unter Stickstoff (16 Nl/h) auf 80°C erwärmt. Danach wird das Pulver bei 80°C über eine Periode von 5 Minuten mit 1,2 g gasförmigem Schwefeltrioxid umgesetzt. Nach Reaktion wird das Pulver 16 Stunden bei 50°C und 100 mbar getrocknet.

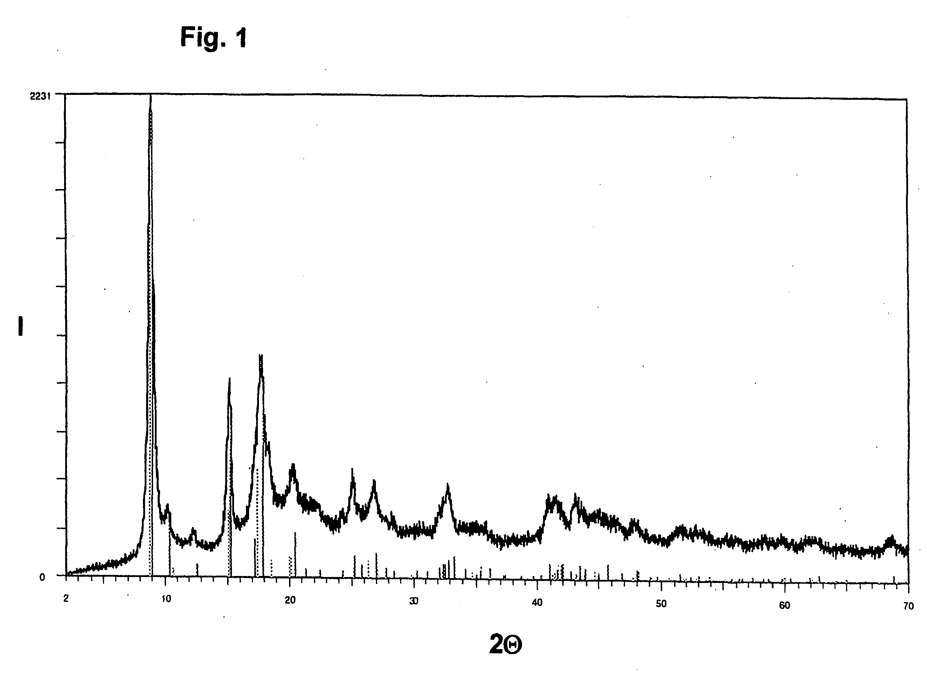

Die Oberfläche (BET) wird zu 494 m²/g bestimmt. Das XRD ist in Fig. 1 dargestellt, wobei I (Lin (counts)) als Funktion von 2 Θ (2-Theta-Skala) gezeigt ist.

Die Elementaranalyse ergibt ein S : C-Verhältnis von 1 : 31. Das Al : S-Verhältnis liegt bei etwa 7 : 1. Daraus ergibt sich rechnerisch eine Säuredichte von ca. 1,0 mmol/g für das sulfonierte Al-MOF-Pulver.

### Beispiel 3 Säurekatalysierte Veresterung von Butanol mit Essigsäure mit Hilfe eines erfindungsgemäßen metallorganischen Gerüstmaterials

In einem 100 ml Dreihalskolben mit Rückflusskühler werden 50 g eines Butanol : Essigsäure (67 : 33 Gew.-%) -Gemisches mit 1,0 g sulfoniertem Al-MOF aus Beispiel 2 versetzt und gerührt. Danach wird das Gemisch auf 75°C erwärmt und nach einer Reaktionsdauer von 6 Stunden eine Probe entnommen. Die Probe wird anschließend gaschromatographisch auf ihre Zusammensetzung untersucht. Diese enthält 7 Flächen-% Essigsäure, 58 Flächen-% Butanol und 35 Flächen-% Butylacetat.

### Vergleichsbeispiel 4 Veresterung von Butanol mit Essigsäure an nicht-sulfoniertem MOF

Der Versuch wird analog zu Beispiel 3 durchgeführt, wobei jedoch hier 1,0 g Al-MOF-Pulver aus Beispiel 1 verwendet wird. Die Probe nach 5,5 Stunden Reaktionszeit enthielt 11 Flächen-% Essigsäure, 73 Flächen-% Butanol und 16 Flächen-% Butylacetat.

## Patentansprüche

1. Poröses metallorganisches Gerüstmaterial enthaltend mindestens eine an mindestens ein Metallion M koordinativ gebundene, mindestens zweizähnige organische Verbindung L, wobei L mindestens eine funktionelle Gruppe G aufweist, die nicht koordinativ an M bindet und ausgewählt ist aus der Gruppe bestehend aus -SO₃H und deren deprotoniertes Analogon, wobei das Gerüstmaterial eine Säuredichte von mindestens 0,1 mmol/g Gerüstmaterial aufweist.

2. Gerüstmaterial nach Anspruch 1, **dadurch gekennzeichnet, dass** das molare Verhältnis G : M mindestens 1 : 75 beträgt.

3. Gerüstmaterial nach Anspruch 1 oder 2, **dadurch gekennzeichnet, dass** dieses eine Säuredichte von mindestens 1 mmol/g Gerüstmaterial aufweist.

4. Gerüstmaterial nach einem der Ansprüche 1 bis 3, **dadurch gekennzeichnet, dass** M ausgewählt ist aus der Gruppe bestehend aus Mg, Ca, Al, Ga, In, Zn, Cu, Fe und Mn.

5. Gerüstmaterial nach einem der Ansprüche 1 bis 4, **dadurch gekennzeichnet, dass** sich L von einer Di-, Tri-, Tetracarbonsäure oder einem Schwefelanalogon oder einem Diamin ableitet.

6. Verfahren zur Herstellung eines Gerüstmaterials nach einem der Ansprüche 1 bis 5, den Schritt enthaltend
- Inkontaktbringen eines Metallions M mit einer gegebenenfalls deprotonierten mindestens zweizähnigen organischen Verbindung L, die mindestens eine funktionelle Gruppe G aufweist, die nicht koordinativ an M bindet und ausgewählt ist aus der Gruppe bestehend aus -SO₃H und deren deprotoniertes Analogon unter Ausbildung des Gerüstmaterials.

7. Verfahren zur Herstellung eines Gerüstmaterials nach einem der Ansprüche 1 bis 5, die Schritte enthaltend
- Inkontaktbringen eines Metallions M mit einer gegebenenfalls deprotonierten mindestens zweizähnigen organischen Verbindung L', die mindestens eine S-haltige Gruppe G' aufweist, die bevorzugt nicht koordinativ an M bindet und
- Umwandlung der Gruppe G' in eine Gruppe G an L.

8. Verfahren nach Anspruch 7, **dadurch gekennzeichnet, dass** G' ein Sulfonat, Sulfit-, Disulfit- Sulfinatgruppe sowie deren Säure, Ester und Halogenid, oder Mercaptogruppe ist.

9. Verfahren zur Herstellung eines Gerüstmaterials nach einem der Ansprüche 1 bis 5, die Schritte enthaltend
- Umsetzung eines porösen metallorganischen Gerüstmaterials enthaltend mindestens eine an mindestens ein Metallion M koordinativ gebundene mindestens zweizähnige organische Verbindung L', welche einen Aromaten oder eine vinylische Doppelbindung enthält, mit einer S-haltigen Verbindung zur Bildung einer Gruppe G an L oder einer Gruppe G' an L und
- sofern G' gebunden ist, Umwandlung von G' zu G.

10. Verwendung eines metallorganischen Gerüstmaterials nach einem der Ansprüche 1 bis 5 als Ionenaustauscher oder Brönstedt-Säure.

## Claims

1. A porous metal organic framework comprising at least one at least bidentate organic compound L coordinated to at least one metal ion M, wherein L has at least one functional group G which bonds noncoordinatively to M and is selected from the group consisting of -SO₃H and its deprotonated analogue, and the framework has an acid density of at least 0.1 mmol/g of framework.

2. The framework according to claim 1, wherein the molar ratio of G:M is at least 1:75.

3. The framework according to claim 1 or 2 which has an acid density of at least 1 mmol/g of framework.

4. The framework according to any of claims 1 to 3, wherein M is selected from the group consisting of Mg, Ca, Al, Ga, In, Zn, Cu, Fe and Mn.

5. The framework according to any of claims 1 to 4, wherein L is derived from a dicarboxylic, tricarboxylic, tetracarboxylic acid or a sulfur analogue or a diamine.

6. A process for preparing a framework according to any of claims 1 to 5, which comprises the step
- contacting of a metal ion M with an optionally deprotonated at least bidentate organic compound L which has at least one functional group G which bonds noncoordinatively to M and is selected from the group consisting of -SO₃H and its deprotonated analogue to form the framework.

7. A process for preparing a framework according to any of claims 1 to 5, which comprises the steps
- contacting of a metal ion M with an optionally deprotonated at least bidentate organic compound L' which has at least one S-comprising group G' which preferably bonds noncoordinatively to M and
- conversion of the group G' into a group G on L.

8. The process according to claim 7, wherein G' is a sulfonate, sulfite, disulfite, sulfinate group or a corresponding acid, ester or halide, or mercapto group.

9. A process for preparing a framework according to any of claims 1 to 5, which comprises the steps
- reaction of a porous metal organic framework comprising at least one at least bidentate organic compound L' which comprises an aromatic or a vinylic double bond and is coordinated to at least one metal ion M with an S-comprising compound to form a group G on L or a group G' on L and
- if G' is present, conversion of G' into G.

10. The use of a metal organic framework according to any of claims 1 to 5 as ion exchanger or Brönsted acid.

## Revendications

1. Matériau de squelette métallo-organique poreux contenant au moins un composé organique L au moins bidentate, relié coordinativement à au moins un ion métallique M, L comportant au moins un groupe fonctionnel G qui ne se relie pas coordinativement à M et qui est choisi dans le groupe constitué par -SO₃H et ses analogues déprotonés, le matériau de squelette présentant une densité d'acide d'au moins 0,1 mmol/g de matériau de squelette.

2. Matériau de squelette selon la revendication 1, **caractérisé en ce que** le rapport molaire G:M est d'au moins 1:75.

3. Matériau de squelette selon la revendication 1 ou 2, **caractérisé en ce que** celui-ci présente une densité d'acide d'au moins 1 mmol/g de matériau de squelette.

4. Matériau de squelette selon l'une quelconque des revendications 1 à 3, **caractérisé en ce que** M est choisi dans le groupe constitué par Mg, Ca, Al, Ga, In, Zn, Cu, Fe et Mn.

5. Matériau de squelette selon l'une quelconque des revendications 1 à 4, **caractérisé en ce que** L dérive d'un acide di-, tri-, tétracarboxylique ou d'un analogue de soufre ou d'une diamine.

6. Procédé de fabrication d'un matériau de squelette selon l'une quelconque des revendications 1 à 5, contenant l'étape de
- mise en contact d'un ion métallique M avec un composé organique L au moins bidentate éventuellement déprotoné, qui comporte au moins un groupe fonctionnel G qui ne se relie pas coordinativement à M et qui est choisi dans le groupe constitué par -SO₃H et ses analogues déprotonés, pour former le matériau de squelette.

7. Procédé de fabrication d'un matériau de squelette selon l'une quelconque des revendications 1 à 5, contenant les étapes de
- mise en contact d'un ion métallique M avec un composé organique L' au moins bidentate éventuellement déprotoné, qui comporte au moins un groupe G' contenant du S, qui ne se relie de préférence pas coordinativement à M, et
- transformation du groupe G' en un groupe G sur L.

8. Procédé selon la revendication 7, **caractérisé en ce que** G' est un groupe sulfonate, sulfite, disulfite, sulfinate, ainsi que son acide, ester et halogénure, ou un groupe mercapto.

9. Procédé de fabrication d'un matériau de squelette selon l'une quelconque des revendications 1 à 5, contenant les étapes de
- réaction d'un matériau de squelette métallo-organique poreux contenant au moins un composé organique L' au moins bidentate relié coordinativement à au moins un ion métallique M, qui contient un composé aromatique ou une double liaison vinylique, avec un composé contenant du S pour former un groupe G sur L ou un groupe G' sur L, et
- si G' est relié, transformation de G' en G.

10. Utilisation d'un matériau de squelette métallo-organique selon l'une quelconque des revendications 1 à 5 en tant qu'échangeur d'ions ou acide de Brönsted.
